# EUROPÄISCHE PATENTANMELDUNG

(11) **EP 3 210 556 A1**
(43) Veröffentlichungstag der Anmeldung: **30.08.2017**
(21) Anmeldenummer: 16157210.2
(22) Anmeldetag: 24.02.2016
(51) Int. Cl.: A61B 18/02

(54) **KOSMETISCHES VERFAHREN ZUR ENTFERNUNG VON TÄTOWIERUNGEN DER MENSCHLICHEN ODER TIERISCHEN HAUT**

(71) Anmelder: Bolz, Daniel, 65623 Hahnstätten (DE); Flaim, Paolo, 39042 Bressanone (IT)
(72) Erfinder: Bolz, Daniel, 65623 Hahnstätten (DE); Flaim, Paolo, 39042 Bressanone (IT)
(74) Vertreter: Weilnau, Carsten

(57) **Zusammenfassung**

Die Erfindung betrifft in einem Aspekt ein Kosmetisches Verfahren zur Entfernung von Tätowierungen (10) der menschlichen oder tierischen Haut (3), wobei ein Vereisungsfluid auf zumindest ein im Bereich der Tätowierung (10) liegendes Flächensegment (10.1, 11.1) von außen auf die Haut (3) aufgebracht wird.

## Beschreibung

### Technisches Gebiet

Die vorliegende Erfindung betrifft ein kosmetisches Verfahren sowie eine zugehörige Vorrichtung zum möglichst narbenfreien Entfernen von Tätowierungen der menschlichen oder der tierischen Haut.

### Hintergrund

Das Versehen einzelner oder großflächiger Hautpartien mit Motiven in Form von Tätowierungen ist geographisch sowie in unterschiedlichsten Kulturkreisen weit verbreitet. Bei Tieren finden Tätowierungen zu Kennzeichnungszwecken statt. Beim Menschen stellen Tätowierungen eine Form der Körpermodifikation dar.

Beim Tätowieren wird die Haut punktiert, wobei gleichzeitig mit einem Durchstechen der sogenannten Oberhaut, der Epidermis, ein Farbmittel in die darunterliegende Hautschicht, d.h. in die die mittlere Hautschicht, in die sogenannte eingelagert wird.

Das nachträgliche Modifizieren und Entfernen von Tätowierungen ist verhältnismäßig aufwendig und geht oftmals mit einer unerwünschten Narbenbildung einher. Mit dem Einsatz verschiedener Laser lassen sich Makrophagen, welche Farbpigmente der Tätowierung innerhalb der Haut einkapseln, aufbrechen. Die in Makrophagen eingeschlossenen Farbpigmente werden mittels der Laserstrahlung durch Lichtabsorption so stark erhitzt, dass sie zerbersten. Es erfolgt jedoch meist eine erneute Einkapselung der Farbpigmente, was eine Wiederholung der Laserbehandlung nach einigen Wochen erforderlich macht. Die mittels Laserbehandlung freigesetzten Farbpigmente werden schließlich vom Körper abgebaut. Gar nicht oder nur schwach abbaubare Farbstoffe können selbst mit einer Laserbehandlung kaum aus der Haut entfernt werden. Ferner können bei der Laserbehandlung von Farbpigmenten krebserregende Substanzen entstehen.

Alternativ zur Laserentfernung existieren Verfahren mit flüssigen Tätowierungs- oder Tattoo-Entfernern. Hierbei handelt es sich meist um Lösungen, die in der Regel 40 % L-(+)-Milchsäure enthalten. Ähnlich wie beim ursprünglichen Einbringen der Tätowierung in die Haut wird hierbei mit einer Nadel unter die Oberhaut gestochen. Die betreffende Lösung wird hierbei ebenfalls in die Dermis eingebracht. Der Einsatz solcher Tattoo-Entfernungsmittel ist aufgrund der Reizwirkung von Milchsäure hoher Konzentration mitunter mit gesundheitlichen Risiken verbunden.

Aus der EP 2 201 904 A1 ist ferner ein Verfahren zur Entfernung von Tätowierungen bekannt, wobei alternierende elektrische Felder zur Erzeugung einer Hochfrequenzentladung zwischen der Epidermis und einer Nadelelektrode erzeugt werden.

Sämtliche bislang bekannten Verfahren zur Entfernung von Tätowierungen der menschlichen oder tierischen Haut sind relativ aufwendig und/oder teuer in ihrer Durchführung und gehen meist mit einer nicht unerheblichen Narbenbildung der betroffenen Hautpartie einher.

Der vorliegenden Erfindung liegt demgegenüber die Aufgabe zugrunde, ein kosmetisches Verfahren sowie eine entsprechende Vorrichtung zur hautschonenden, möglichst narbenfreien Entfernung von Tätowierungen der menschlichen oder tierischen Haut zur Verfügung zu stellen. Das Verfahren soll besonders einfach und kostengünstig sowie hautschonend durchführbar sein. Für Betroffene und für Endanwender sollen das Verfahren und die zugehörige Vorrichtung ein hohes Maß an Anwendersicherheit bereitstellen, sodass auch Personen mit nur geringer medizinischer Schulung grundsätzlich zur anwendersicheren Durchführung des Verfahrens infrage kommen. Die Anschaffungs- und Investitionskosten für eine zur Durchführung des Verfahrens geeigneten Vorrichtung sollen möglichst gering sein. Zudem soll die Durchführung des Verfahrens frei von jeglicher Gesundheitsgefährdung sowohl für den Anwender als auch für den Betroffenen sein.

Diese Aufgabe wird mit einem kosmetischen Verfahren zur Entfernung von Tätowierungen der menschlichen oder tierischen Haut gemäß dem unabhängigen Patentanspruch 1 sowie mit einer zugehörigen Vorrichtung zur Durchführung des Verfahrens gemäß dem unabhängigen Patentanspruch 10 gelöst. Vorteilhafte Ausgestaltungen sind dabei jeweils Gegenstand abhängiger Patentansprüche.

### Zusammenfassung der Erfindung

Das demgemäß vorgesehene Verfahren ist zur Entfernung von Tätowierungen der menschlichen oder tierischen Haut ausgestaltet. Das Verfahren besteht darin, ein Vereisungsfluid auf zumindest ein im Bereich der Tätowierung der Haut liegendes Flächensegment von außen auf die Haut aufzubringen. Das lokale Aufbringen eines Vereisungsfluids in einem vorgegebenen Flächensegment führt zu einer lokalen Vereisung der Haut im Bereich des besagten Flächensegments. Die betroffene Hautschicht, in welcher die Farbpigmente der Tätowierung liegen, wird hierbei punktuell gefroren bzw. eingefroren. Die Haut stirbt im Bereich jenes Flächensegments lokal ab. Dieses Absterben regt die Neubildung von darunterliegenden Hautzellen an, sodass die abgestorbenen Hautzellen mit der darin eingeschlossenen oder eingekapselten Farbe nach außen aus dem Körper herausgedrängt werden.

Dies geschieht indem nach der Vereisungsbehandlung die im Bereich des behandelten Flächensegments abgestorbene Haut und in diesem Bereich vorhandene Farbpigmente in einer vom Körper gebildeten Kruste gebunden werden und infolge der Neubildung von Hautzellen aus dem Körper sozusagen herauswachsen. Nachdem der durch die lokale Vereisung getriggerte lokale Neubildungsprozess von Hautzellen abgeschlossen ist, fällt die Kruste mit den darin eingekapselten Farbpigmenten von selbst ab. Mittels der lokalen Vereisung von Hautzellen kann auf diese Art und Weise eine überaus schonende und gesundheitlich unbedenkliche sowie vollständige Entfernung von Farbpigmenten aus der Haut erreicht werden. Das Verfahren ist besonders sensitiv und hautregenerierend.

Das Aufbringen des Vereisungsfluids ist sowohl für eine von der Tätowierung betroffene Person oder ein Tier als auch für den Anwender, welcher das Vereisungsfluid auf das Flächensegment aufbringt, gesundheitlich vollkommen unbedenklich. Es ist lediglich auf die Einhaltung ohnehin vorgegebener Hygienestandards im kosmetischen Bereich zu achten. Das punktuelle und lokale Vereisen oder Einfrieren von Flächensegmenten der Haut kann ferner vergleichsweise zügig erfolgen, sodass eine effektive Entfernung der Tätowierung bereits bei einer vergleichsweise kurzen Behandlungsdauer möglich wird.

Nach einer Weiterbildung ist vorgesehen, dass vor einem Aufbringen des Vereisungsfluids auf die Haut das betreffende Flächensegment der Haut einer kosmetischen Dermabrasion unterzogen wird. Es ist denkbar, den gesamten Bereich der Tätowierung großflächig einer Dermabrasion zu unterziehen. Es ist aber auch denkbar, lediglich einzelne flächenmäßig begrenzte Flächensegmente der Haut einer kosmetischen Dermabrasion zu unterziehen, wobei nur solche Flächensegmente der Haut im Bereich der Tätowierung einer Dermabrasion unterzogen werden, welche später auch mit dem Vereisungsfluid behandelt werden. Mittels einer kosmetischen Dermabrasion wird die oberste Hautschicht, das heißt die Oberhaut oder die Epidermis wenigstens bereichsweise geöffnet und/oder zumindest partiell abgetragen. Durch eine kosmetische Dermabrasion wird die Dermis zumindest bereichsweise, bevorzugt nur minimal keinesfalls aber bis zum darunterliegenden sogenannten Stratum Papillare abgetragen.

Es ist zwar grundsätzlich denkbar, die Epidermis in einzelnen Bereichen vollständig oder annähernd vollständig abzutragen. Auf diese Art und Weise kann das Vereisungsfluid unmittelbar auf die mit den Farbpigmenten versehene Dermis aufgebracht werden bzw. unmittelbar mit der Dermis in Kontakt gelangen. Die mit den Farbpigmenten versehene Hautschicht kann auf diese Art und Weise gut und lokal begrenzt vereist bzw. auf eine Temperatur unterhalb der Zerstörschwelle des betreffenden Körpergewebes abgekühlt werden.

Aber auch ein minimaler Abtrag der Epidermis oder ein bloßes Öffnen der Epidermis ist für die Verbesserung einer Kühlwirkung und für ein geometrisch lokal begrenztes Aufbringen des Vereisungsfluids bereits von Vorteil. Ein möglichst geringer Abtrag der Epidermis erweist sich allgemein als hautschonend.

Das Öffnen und/oder bereichsweise Entfernen der Epidermis mittels kosmetischer Dermabrasion verbessert den Kühleffekt des Vereisungsfluids an und in der mit Farbpigmenten versehenen Hautschicht. Es wird hierdurch prinzipiell ermöglicht, bei einer erstmaligen Anwendung des kosmetischen Verfahrens sämtliche im Bereich des betroffenen Flächensegments vorhandenen Farbstoffe im Zuge der Hautneubildung und Krustenbildung aus der Haut nach außen zu transportieren und somit wirksam aus der Haut zu entfernen.

Nach einer weiteren Ausgestaltung wird eine oberste Hautschicht mittels der Dermabrasion lokal geöffnet oder bis zu einer maximalen Tiefe von weniger als 0,1 mm oder von weniger als 0,05 mm abgetragen. Ein derart geringer Abtrag der obersten Hautschicht, sprich der Epidermis, kann mithin auch als Peeling bezeichnet werden. Es ist insbesondere vorgesehen, dass das mit dem Vereisungsfluid zu behandelnde Flächensegment der Haut vorab, das heißt vor Aufbringen des Vereisungsfluids einer sogenannten Mikrodermabrasion unterzogen wird. Hierbei handelt es sich um eine kontrollierte, mechanische Abtragung der obersten Hautschicht(en). Die bereits beschriebene Dermabrasion kann insbesondere als Mikrodermabrasion durchzuführen sein.

Die Mikrodermabrasion ist als ein rein kosmetisches Behandlungsverfahren einzustufen, welches für behandelnde Personen, etwa für einen Kosmetiker oder eine Kosmetikerin lediglich eine vergleichsweise kurze Einarbeitungszeit erfordert. Eine Mikrodermabrasion kann nach geltenden Vorschriften von einem Kosmetiker durchgeführt werden, da es sich hierbei um eine rein kosmetische Prozedur handelt.

Die Mikrodermabrasion kann mittels Schleifgeräten oder auch durch Bestrahlung mit kleinen Kristallen, etwa Aluminiumoxid, Salz oder mikrofeinem Sand erfolgen, welcher im Zuge der Behandlung auch wieder abgesaugt wird. In Grundzügen kann die Mikrodermabrasion einem Sandstrahlen entsprechen. Lokale Hautabschleifungen können hierbei ohne chemische Wirkstoffe erfolgen. Zugleich wird das betroffene Flächensegment der zu behandelnden Haut mittels der kosmetischen Dermabrasion bzw. mittels der Mikrodermabrasion besonders gut gereinigt. Mittels kosmetischer Dermabrasion oder Mikrodermabrasion werden ferner auch ohnehin bereits abgestorbene Hautzellen völlig schmerzfrei entfernt.

Zudem kann mittels der Dermabrasion oder Mikrodermabrasion auch die Hautneubildung angeregt werden. Dies kann sich für die Krustenbildung und für das Herauswachsen der Kruste aus dem Bereich der Dermis als vorteilhaft erweisen. Mittels der kosmetischen Dermabrasion oder Mikrodermabrasion soll die oberste oder äußerste Hautschicht, das heißt die Epidermis höchstens um 0,1 mm oder sogar höchstens um 0,05 mm abgetragen werden. Der Abtrag der Epidermis soll so schonend als möglich erfolgen. Keinesfalls sollen die unterhalb der Epidermis liegenden Hautschichten einer direkten mechanischen Beanspruchung oder Verletzungen unterzogen werden. Es ist besser, wenn die Epidermis nur bereichsweise und nicht vollständig abgetragen wird, als dass im Zuge der Dermabrasion die Epidermis vollständig und darunterliegende Hautschichten, so etwas das Stratum Papillare mechanisch verletzt oder mechanisch beschädigt würden.

Nach einer weiteren Ausgestaltung des kosmetischen Verfahrens ist vorgesehen, dass die maximale Größe des mit dem Vereisungsfluid zu versehenen Flächensegmentes im Bereich der Tätowierung zwischen 5 mm² und 20 mm² beträgt. Die zu behandelnden Flächensegmente können beispielsweise kreisförmig oder elliptisch ausgestaltet sein. Es sind aber auch beliebige andere geometrische Konturen für die mit dem Vereisungsfluid zu behandelnden Flächensegmente denkbar. Bei einer kreisrunden Ausgestaltung des mit dem Vereisungsfluid zu behandelnden Flächensegments kann dieses einen maximalen Durchmesser von 2,5 mm bis etwa 5 mm aufweisen.

Derart kleine Flächensegmente ermöglichen einen guten und für den Betroffenen kaum wahrnehmbaren Krustenbildungsprozess. Ferner können etwaige und infolge der Krustenbildung entstehende Oberflächenspannungen in der betroffenen Haut auf ein Minimum reduziert werden. Hierdurch kann ferner einem Aufreißen der bereits gebildeten Kruste effektiv entgegengewirkt werden. Derart kleine Flächensegmente schränken ferner die Beweglichkeit der betroffenen Person im Bereich der Tätowierung kaum ein.

Nach einer Weiterbildung des kosmetischen Verfahrens wird ein mit der Tätowierung versehener Hautbereich in mehrere Flächensegmente unterteilt. Es werden hierbei nur solche Flächensegmente mit dem Vereisungsfluid beaufschlagt, die um einen vorgegebenen Abstand voneinander entfernt sind. Es werden insbesondere nur solche Flächensegmente gleichzeitig oder unmittelbar nacheinander mit dem Vereisungsfluid beaufschlagt, die kontaktfrei zueinander angeordnet sind. Die mit dem Vereisungsfluid zu behandelnden Flächensegmente weisen typischerweise einen Abstand zueinander auf, der in etwa zumindest in der gleichen Größenordnung wie der Durchmesser oder die Fläche der Flächensegmente selbst ist.

Solche Flächensegmente, die in einer ersten Behandlung nicht mit dem Vereisungsfluid behandelt wurden, werden im Zuge einer Wiederholung der gesamten Prozedur in einer ähnlichen oder identischen Art und Weise wie bereits in Rahmen einer ersten Prozedur behandelte Flächensegmente zu behandeln sein. Eine zweite, d.h. wiederholte Behandlung der Haut im Bereich der Tätowierung erfolgt erst, nachdem die im Zuge einer ersten und vorangegangenen Behandlung behandelte Haut vollständig abgeheilt ist, das heißt, nachdem sämtliche infolge der Vereisung gebildeten Krusten abgefallen sind.

Je nach Art der zu entfernenden Farbpigmente und der Lage der Pigmente innerhalb der Haut kann ohnehin die Wiederholung des beschriebenen kosmetischen Verfahrens in regelmäßigen Abständen erforderlich werden. Da jede einzelne Behandlung mit dem Vereisungsfluid weitgehend narbenfrei verlaufen kann, kann sich in Summe und infolge einer mehrfachen Durchführung des hier beschriebenen Verfahrens auch eine vollständig narbenfreie Entfernung von Tätowierungen der menschlichen oder tierischen Haut ergeben.

Nach einer weiteren Ausgestaltung des Verfahrens ist zur Einteilung der Haut in Flächensegmente und zur Applikation des Vereisungsfluids eine auf die Haut auflegbare Maske mit Durchgangsöffnungen vorgesehen. Die besagte Maske wird hierbei auf die Haut aufgelegt. Die Durchgangsöffnungen geben dabei diejenigen Flächensegmente der Haut an, welche mit dem Vereisungsfluid beaufschlagbar sind. Die Geometrie und Position der Durchgangsöffnungen stimmt hierbei mit der Geometrie und der Position der zu behandelnden Flächensegmente der Haut überein. Optional legen die in der Maske vorgesehenen Durchgangsöffnungen auch diejenigen Flächensegmente der Haut fest, welche der kosmetischen Dermabrasion vor Applizierung des Vereisungsfluids unterzogen werden.

Die Maske kann als flexible Maske, beispielsweise in Form einer flexiblen Folie oder Schale ausgestaltet sein. Ferner ist die Maske reibschlüssig oder stoffschlüssig mit der zu behandelnden Haut fixierbar. Die Maske kann beispielsweise mit geeigneten Gurten oder aber mittels Klebestreifen außen an der Haut befestigt und fixiert werden.

Die Maske kann ferner eine thermisch isolierende Wirkung bereitstellen. Ferner kann die Maske auch aus einem thermisch isolierenden Material, beispielsweise aus einem thermoplastischen und/oder elastischen Kunststoff gefertigt sein, welcher selbst bei tiefen Temperaturen keine Rissbildung zeigt und der insoweit tieftemperaturstabil ist. Insoweit können die durch die Maske abgedeckten und außerhalb der Durchgangsöffnungen der Maske liegenden Bereiche der Haut gegen Kälte geschützt werden. Die Maske kann ferner einen oder mehrere thermoplastische Elastomerwerkstoffe, wie z.B. natürliche oder synthetische Gummiwerkstoffe aufweisen oder hieraus bestehen.

Nach einer weiteren Ausgestaltung liegt das Vereisungsfluid in Form einer kryogenen Flüssigkeit, eines kryogenen Gases oder Form eines kryogenen Flüssigkeits-GasGemischs vor. Das Vereisungsfluid, das heißt die kryogene Flüssigkeit, das kryogene Gas oder ein entsprechendes kryogenes Flüssigkeits-Gas-Gemisch, beispielsweise auch in Form eines Aerosols, wird auf das zumindest eine Flächensegment der Haut aufgebracht. Das Vereisungsfluid kann beispielsweise in Tropfenform und mit Hilfe der Maske lokal begrenzt auf die Haut aufgebracht werden. Ferner ist denkbar, das Vereisungsfluid auf das betreffende Flächensegment der Haut aufzusprühen. Es ist insbesondere vorgesehen, das Vereisungsfluid mittels eines besonders feinen und fokussierten Strahls auf das Flächensegment zu richten.

Als Vereisungsfluid kommen unterschiedliche Stoffe und Stoffgemische, insbesondere flüssiger Stickstoff sowie flüssige Stickstoffverbindungen, so zum Beispiel Distickstoffmonoxid (N₂O) infrage. Derartige als Vereisungsfluid fungierte Kryoflüssigkeiten sind kommerziell in großer Menge und recht kostengünstig erhältlich. Distickstoffmonoxid ist beispielsweise in Form vergleichsweise handlicher Druckbehälter oder Kartuschen erhältlich.

Dass Vereisungsfluid kann in Form eines sogenannten tiefkalten Gases oder tiefkalten Gasgemischs zur Vereisung eingesetzt werden. Es können hierbei Arbeitstemperaturen zwischen 228 K (-45 C) und 77 K (-196 C) angewendet werden. Vereisungen der Haut für den hier beschriebenen Zweck können schon mit Temperaturen unterhalb von -49°C erreicht. Die nötigen tiefen Temperaturen können durch Entspannung hochkomprimierter Gase erreicht werden. Mit Distickstoffmonoxid sind Temperatur von 184,4 K bzw. -88,8 . Mit Kohlendioxid (CO₂) können Temperaturen von 194,7 K bzw. - 78,5 C erzielt werden. Tiefkalt verflüssigter Stickstoff hingegen siedet drucklos bei 77 K bzw. -196°C.

Nach einer weiteren Ausführungsform ist vorgesehen, dass ein Flächensegment der Haut für eine Zeitdauer von höchstens 15 Sekunden, höchstens 20 Sekunden oder von höchstens 30 Sekunden mit dem Vereisungsfluid beaufschlagt wird. Eine zeitliche Begrenzung der Behandlung des Flächensegments mit dem Vereisungsfluid beugt etwaigen großflächigen Verletzungen der Haut vor. Typische Applikationszeiten im Bereich von 15 bis 20 Sekunden sind meist ausreichend, um die mit den Farbpigmenten versehene Hautschicht(en) im Bereich der Dermis kontrolliert absterben zu lassen.

Nach einer weiteren Ausgestaltung ist vorgesehen, dass das Vereisungsfluid in einer kreisenden unterbrochenen Bewegung über das gesamte ausgewählte Flächensegment der Haut verteilt wird. Dies trifft insbesondere für ein in Form eines gerichteten Strahls auf das Flächensegment applizierten Vereisungsfluids zu. Bei der Beaufschlagung des betroffenen Flächensegments mit dem Vereisungsfluid soll die Bildung einer Eisschicht möglichst vermieden werden. Durch eine kreisende Bewegung beim Aufsprühen des Vereisungsfluids kann mittels eines konzentrierten Vereisungsfluidstrahls einer Eisschichtbildung effektiv entgegengewirkt werden. Sich unweigerlich auf der Hautoberfläche bildende Eiskristalle können mittels der Strahlwirkung des aufgebrachten Fluids mechanisch entfernt werden. Ferner kann mittels einer kreisenden ununterbrochenen Bewegung das mit dem Vereisungsfluid zu beaufschlagende Flächensegment möglichst gleichmäßig und homogen einer Kältebehandlung unterzogen werden. Es ist hierbei von Vorteil, den Strahl unter einem vorgegebenen Neigungswinkel von bis zu 30° gegenüber einer Flächennormalen der zu behandelnden Hautpartie auf das Flächensegment zu richten.

Nach einem weiteren Aspekt betrifft die Erfindung ferner eine Vorrichtung zur Durchführung des zuvor beschriebenen kosmetischen Verfahrens zur Entfernung von Tätowierungen der menschlichen oder tierischen Haut. Die Vorrichtung umfasst einen Applikator zum Aufbringen eines Vereisungsfluids auf ein im Bereich der Tätowierung liegendes Flächensegment von außen auf die Haut. Die Vorrichtung umfasst ferner eine auf die Haut auflegbare und mit mehreren Durchgangsöffnungen versehene Maske.

Mittels der Maske können einzelne der Kältebehandlung zu unterziehende Flächensegmente der Haut bestimmt und festgelegt werden. Alsdann können sämtliche im Bereich der Tätowierung liegende Durchgangsöffnungen der Maske mit dem Vereisungsfluid beaufschlagt werden. Dies hat zur Folge, dass die unmittelbar von den Durchgangsöffnungen freigelegten und über die Durchgangsöffnungen zugänglichen Flächensegmente der Haut lokal begrenzt mit dem Vereisungsfluid in Kontakt gelangen. Ein Mindestabstand benachbarter Durchgangsöffnungen liegt im Bereich der Größe, das heißt des Durchmessers oder der Fläche der Durchgangsöffnungen. Der Abstand benachbarter Durchgangsöffnungen kann aber auch das 1,5- bis 3-Fache des Durchmessers oder Fläche der Durchgangsöffnungen betragen. Typischerweise sind die Durchgangsöffnungen äquidistant und gleichmäßig verteilt über der Fläche der Maske angeordnet. Sämtliche Durchgangsöffnungen weisen typischerweise identische Konturen oder Geometrien auf.

Die Maske ist ferner mittels geeigneten Befestigungselementen an der Haut befestigbar bzw. hieran fixierbar. Auf diese Art und Weise kann die Maske sowohl eine lokal begrenzten Dermabrasion der darunterliegenden und über die Durchgangsöffnungen zugänglichen Hautsegmente als für eine lokal begrenzte nachfolgende Kältebehandlung dienen.

Die Maske erfüllt insoweit mehrere Zwecke, sie dient der Einteilung der Haut in einzelnen Flächensegmente und gibt insoweit ein Behandlungsraster für eine Behandlungsprozedur vor. Gleichzeitig dient die Maske einer lokalen Begrenzung der kosmetischen Anwendung auf die ausgewählten Hautsegmente, nämlich der Begrenzung der Dermabrasion als auch der Vereisungs- oder Kryobehandlung.

Nach einer weiteren Ausgestaltung weist die Vorrichtung ferner ein Dermabrasionsgerät auf, um das der Kältebehandlung zu unterziehende Flächensegment, bzw. um mehrere einer Kältebehandlung zu unterziehende Flächensegmente vorab einer kosmetischen Dermabrasion zu unterziehen. Insbesondere kann die Vorrichtung ein Behandlungskit für eine schonende, möglichst narbenfreie Entfernung von Tätowierungen der menschlichen oder tierischen Haut bereitstellen. Das Behandlungskit kann dabei aus einem Applikator zum Aufbringen des Vereisungsfluids, einer mit Durchgangsöffnungen versehenen Maske und einem Dermabrasionsgerät bestehen.

Der Applikator kann insbesondere als tragbarer Applikator ausgestaltet sein, welcher sich durch eine Einhandbetätigung auszeichnet. Der Applikator kann stiftförmig ausgestaltet sein. An seinem distalen Ende kann er eine Strahlöffnung aufweisen, über welche ein konzentrierter Strahl des Vereisungsfluids auf eine betroffene Stelle der Haut gerichtet werden kann. An einem gegenüberliegenden Ende kann der Applikator einen abnehmbaren Gehäusedeckel aufweisen, welcher eine Einführöffnung für eine Kartusche aufweist. In jene Einführöffnung können beispielsweise unter Druck stehende und mit einem gasförmigen und/oder flüssigen Vereisungsfluid, bzw. Kryofluid gefüllte Kartuschen eingesetzt werden. Das im Inneren der Kartusche herrschende Druckniveau kann für die Erzeugung eines Strahls und für das selbsttätige Austreten eines Strahls der Kryoflüssigkeit verwendet werden. Insoweit kann der Applikator rein druckbetrieben und stromlos ausgestaltet sein.

### Kurzbeschreibung der Figuren

Weitere Ziele, Merkmale sowie vorteilhafte Ausgestaltungen des kosmetischen Verfahrens und der Vorrichtung zur Durchführung desselben werden in der nachfolgenden Beschreibung anhand eines Ausführungsbeispiels exemplarisch dargestellt. Hierbei zeigen:
- Fig. 1: eine mit einer Tätowierung versehene Körperpartie,
- Fig. 2: eine gedachte Segmentierung der Tätowierung in einzelne Flächensegmente,
- Fig. 3: eine Unterteilung der Tätowierung in einzelne Flächensegmente mit Hilfe einer Maske,
- Fig. 4: ein Flussdiagramm des kosmetischen Verfahrens und
- Fig. 5: die einzelnen Komponenten der zur Durchführung des Verfahrens vorgesehenen Vorrichtung.

### Detaillierte Beschreibung

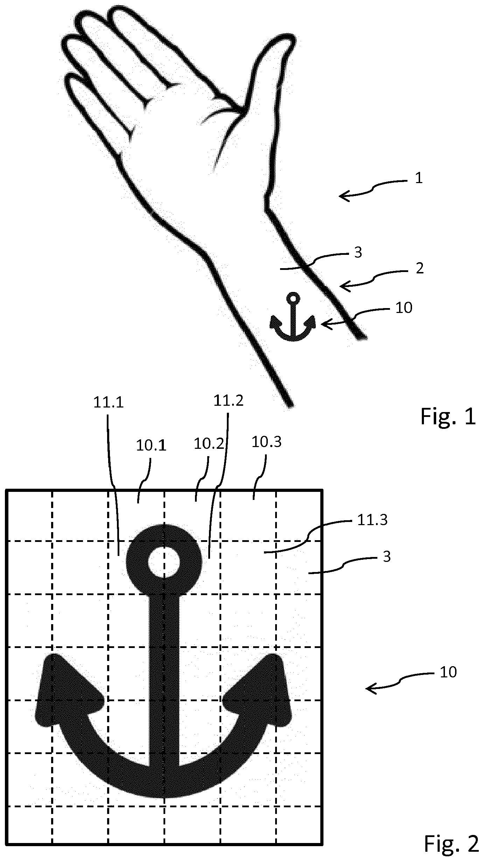

In Fig. 1 ist ein Körperteil 1 des menschlichen Körpers, nämlich ein Unterarm 2 gezeigt. Die Haut 3 des Unterarms 2 ist mit einer Tätowierung 10 versehen. Die die Tätowierung bildenden Farbpigmente befinden sich typischerweise unterhalb der Epidermis der Haut 3, typischerweise innerhalb der sogenannten Dermis, etwa im Stratum Papillare.

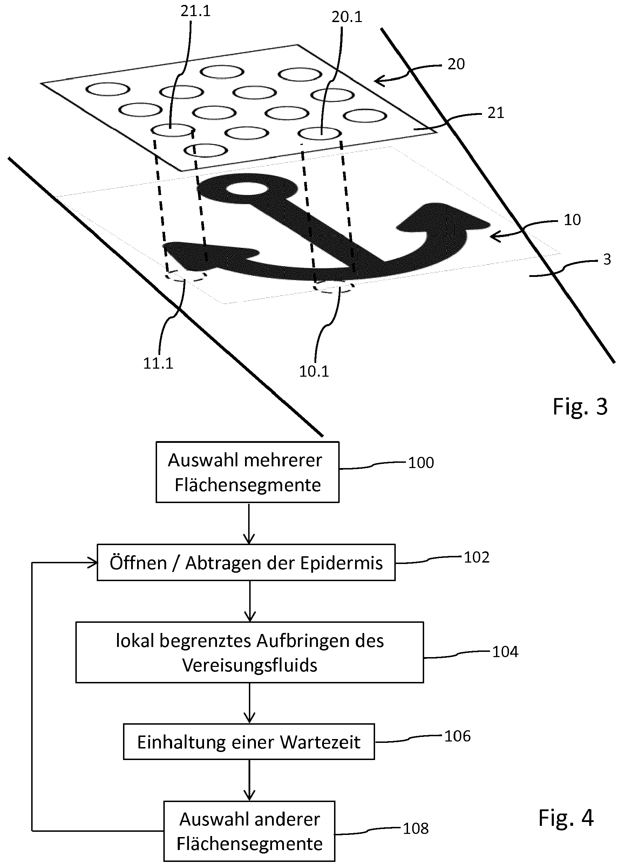

Zur Entfernung der Tätowierung 10 ist nach dem erfindungsgemäßen kosmetischen Verfahren das Aufbringen eines Vereisungsfluids, insbesondere einer kryogenen Flüssigkeit, eines kryogenen Gases oder eines kryogenen Flüssigkeits-Gas-Gemischs vorgesehen. Es werden hierbei nur einzelne Flächensegmente der Haut 3 mit dem Vereisungsfluid, beispielsweise mit flüssigem Distickstoffmonoxid beaufschlagt. Vor der kosmetischen Behandlung wird die Haut 3 im Bereich der Tätowierung 10 in einzelne Flächensegmente 10.1, 10.2, 10.3, 11.1, 11.2, 11.3 usw. unterteilt, wie dies beispielhaft in Fig. 2 dargestellt ist. Hier wird eine quadratische oder rechteckige Unterteilung in einzelne Flächensegmente gezeigt. Es können aber auch, so wie beispielsweise in Fig. 3 dargestellt, kreisrunde oder ovale Flächensegmente 10.1, 11.1 für die Kryobehandlung der Haut 3 ausgewählt werden.

Für eine gleichzeitige bzw. unmittelbar aufeinanderfolgende Kältebehandlung einzelner Flächensegmente 10.1, 11.1 auf der Haut 3 können die betroffenen Flächensegmente 10.1, 11.1 entweder mittels eines temporären und abwaschbaren Farbauftrags markiert werden. Alternativ ist die Verwendung einer Maske 20 mit mehreren Durchgangsöffnungen 20.1, 21.1 vorgesehen. Die Maske 20 weist typischerweise einen planaren Maskenkörper 21 mit mehreren Durchgangsöffnungen 20.1, 21.1 auf. Die Maske 20 ist zur direkten Auflage auf die Haut 3 vorgesehen. Sie kann mittels Gurten und/oder mittels Klebestreifen ggf. auch mittels einer Klebebeschichtung an ihrer Unterseite an der betroffenen Hautpartie 3 befestigt und hieran fixiert werden.

Der Abstand der einzelnen Durchgangsöffnungen 20.1, 21.1, insbesondere der Abstand unmittelbar benachbart im Maskenkörper 21 vorgesehener Durchgangsöffnungen 20.1, 21.1 ist typischerweise mindestens so groß wie der Durchmesser oder der Querschnitt durch eine der Durchgangsöffnungen 20.1, 21.1. Vorzugsweise ist der Abstand benachbarter Durchgangsöffnungen 20.1, 21.1 mindestens 1,5-mal so groß, 2-mal so groß oder zumindest 3-mal so groß wie der Durchmesser der einzelnen Durchgangsöffnungen 20.1, 21.1. Bei einer direkten Auflage auf die Haut 3 definieren die Durchgangsöffnungen 20.1, 21.1 unmittelbar darunterliegende Flächensegmente 10.1, 11.1 der Haut 3.

Für die Behandlung werden natürlich nur solche Flächensegmente 10.1, 11.1 ausgewählt, die mit der Tätowierung 10 versehen sind.

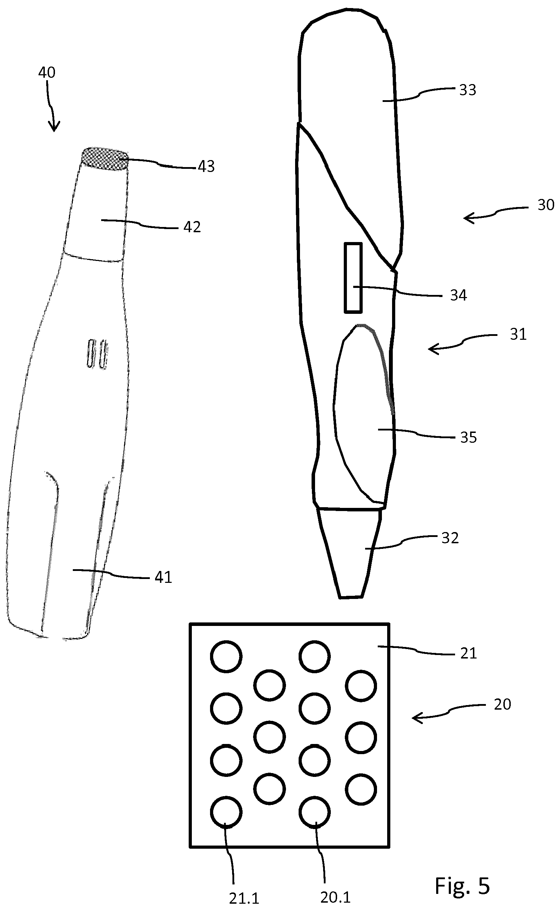

Die beispielsweise mittels der Maske 20 markierten Flächensegmente 10.1, 11.1 der Haut 3 werden mit dem tiefkalten kryogenen Fluid für einen vorgegebenen Zeitraum behandelt. Typischerweise wird mittels eines in Fig. 5 gezeigten Applikators 30 das kryogene Fluid bzw. das Vereisungsfluid punktuell und ausschließlich auf die gekennzeichneten Flächensegmente 10.1, 11.1 der Haut 3 aufgebracht. Die Behandlung einzelner Flächensegmente 10.1, 11.1 mit dem Vereisungsfluid bzw. mit dem kryogenen Fluid beträgt typischerweise 15 bis maximal 30 Sekunden.

Durch lokales und räumlich begrenztes Aufbringen der kryogenen Flüssigkeit bzw. des Vereisungsfluids werden die betroffenen Stellen der Haut vereist bzw. derart stark gekühlt, dass das betroffene Gewebe abstirbt. Der menschliche oder tierische Körper wird hierdurch angeregt, neue Hautzellen zu bilden und die abgestorbenen und mit den Farbpigmenten versehenen Hautzellen mit der darin eingeschlossenen Farbe nach außen aus dem Körper zu transportieren. Die abgestorbenen Hautzellen und die darin eingeschlossenen Farbpigmente werden dabei in einer Kruste eingebunden, die sukzessive aus der Haut bzw. aus dem Körper der betroffenen Person bzw. des betroffenen Tiers herauswächst.

Der Applikator 30 weist typischerweise ein Gehäuse 31 auf. Das längserstreckte, typischerweise stift- oder stabförmige Gehäuse 31 weist an einem longitudinalen bzw. distalen Ende eine Ausströmdüse 32 für die kryogene Flüssigkeit auf. Mittels der Düse 32 kann ein fokussierter bzw. konzentrierter Strahl kryogener Flüssigkeit recht präzise auf das zuvor ausgewählte Flächensegment 10.1, 11.2 der Haut 3 aufgebracht werden. Mittels eines konzentrierten Strahls der kryogenen Flüssigkeit kann zudem eine Eisbildung auf der Hautoberfläche 3 vermieden werden. Der Impuls des Strahls kann derart groß sein, dass infolge der Kryobehandlung gebildete Eispartikel auf der Hautoberfläche 3 im Bereich der Durchgangsöffnungen 20.1, 21.1 etwa durch eine kreisende und unter Umständen leicht gegenüber der Vertikalen geneigten Bewegung des Strahls weggesprüht werden können. Das Aufbringen der kryogenen Flüssigkeit mittels einer kreisenden Bewegung innerhalb des für die Behandlung ausgewählten Flächensegments 10.1, 11.1 dient ferner einem gleichmäßigen Auftrag der kryogenen Flüssigkeit.

Von Vorteil ist vor der Kältebehandlung ausgewählter Flächensegmente 10.1, 11.1 der Haut 3 die Epidermis im Bereich der mittels der Maske 20 ausgewählten Flächensegmente 10.1, 11.1 entweder zu öffnen oder zumindest bereichsweise zu entfernen. Hierfür kann ein Dermabrasionsgerät 40 verwenden werden. Ein in Fig. 5 beispielhaft gezeigtes Dermabrasionsgerät 40 weist typischerweise ein längserstrecktes Gehäuse 41 auf. An einem longitudinalen Ende des Gehäuses 41, beispielsweise an einem distalen Ende, kann ein Aufsatz 42 mit einem Abrasionswerkzeug 43 auswechselbar angeordnet sein. Das Dermabrasionsgerät 40 kann insbesondere in Form eines Mikrodermabrasionsgeräts ausgestaltet sein.

Je nach Einsatz- und Verwendungszweck und je nach Größe der zu behandelnden Flächensegmente 10.1, 11.1 kann ein jeweils geeigneter Aufsatz 42 Verwendung finden. Das Abrasionswerkzeug 43 kann beispielsweise als rotierendes Schleifwerkzeug ausgestaltet sein. Es kann eine für eine Schleifwirkung geeignete vergleichsweise raue Oberfläche aufweisen, um die äußerste Hautschicht zumindest zu öffnen oder bereichsweise abzutragen. Die Epidermis wird hierbei typischerweise nur bis zu einer maximalen Tiefe von weniger als 0,1 mm oder von weniger als 0,05 mm abgetragen.

Das gezielte und vorsichtige Öffnen bzw. Abtragen der Epidermis ermöglicht einen verbesserten, gegebenenfalls auch unmittelbaren thermischen Kontakt des Vereisungsfluids mit der darunterliegenden Dermis oder dem Stratum Papillare. Durch lokales Öffnen oder Beseitigen der Epidermis kann ein besonders guter und unmittelbarer Kälteeintrag in die darunterliegende Dermis bzw. in das Stratum Papillare erfolgen. Eine ansonsten etwa thermisch isolierende Wirkung der Epidermis kann auf diese Art und Weise verringert oder gänzlich beseitigt werden. Der minimale Abtrag bzw. das Öffnen der Epidermis ist ferner weitgehend schmerzfrei. Insbesondere bei Durchführung einer sogenannten Mikrodermabrasion gleicht das Öffnen bzw. das bereichsweise Abtragen der Epidermis einer Art Peeling.

Anstelle eines rotierenden Abrasionswerkzeugs kann die betroffene Hautpartie auch beispielsweise mit kleinen Kristallen, etwa mit Aluminiumoxid, Salz oder mit mikrofeinem Sand abgestrahlt werden.

Das bereichsweise Öffnen bzw. Abtragen der Epidermis geht ferner mit einem Reinigungseffekt einher. Ferner wird durch die Dermabrasion oder Mikrodermabrasion die Neubildung von Hautzellen angeregt. Der Heilungsprozess nach Durchführung der lokalen Kältebehandlung kann auf diese Art und Weise weitaus schneller und zügiger ablaufen.

Das kosmetische Verfahren zur Entfernung von Tätowierungen gliedert sich somit in diverse Verfahrensschritte, wie diese schematisch im Flussdiagramm gemäß Fig. 4 dargestellt sind. In einem ersten Schritt 100 werden einzelnen Flächensegmente der Haut 3 im Bereich der Tätowierung für die Behandlungsprozedur ausgewählt oder bestimmt. Hierzu kann die bereits beschriebene Maske 20 auf die Haut aufgelegt und hieran fixiert werden. Die Maske 20 mit ihren Durchgangsöffnungen 20.1, 21.1 bestimmt dabei ein Behandlungsraster. Anschließend wird im Schritt 102 die Epidermis eines ausgewählten Flächensegments 10.1 der Haut 3 geöffnet oder mittels Dermabrasion, insbesondere mittels Mikrodermabrasion zumindest partiell, ggf. auch vollständig abgetragen. Hiernach wird im Schritt 104 das Vereisungsfluid, typischerweise in Form des bereits beschriebenen kryogenen Fluids lediglich und ausschließlich auf die ausgewählten Flächensegment 10.1, 11.1 aufgebracht. Dies erfolgt typischerweise unter Zuhilfenahme des Applikators 30. Hierbei wird insbesondere ein feiner bzw. fokussierter und räumlich eng begrenzter Strahl des kryogenen Fluids in kreisenden Bewegungen möglichst gleichmäßig über das betroffene Flächensegment 10.1 verteilt. Einzelne Flächensegmente 10.1, 11.1 weisen typischerweise eine Größe zwischen 5 mm² und 20 mm² auf. Bei kreisrunden Flächensegmenten 10.1, 11.1 sind Durchmesser zwischen 2,5 mm und 5 mm vorgesehen. Derart kleine Flächensegmente ermöglichen einen weitreichend spannungsfreien Heilungsprozess der Haut 3.

Nach der Kältebehandlung im Schritt 104 ist in einem nachfolgenden Schritt 106 eine individuell variierende Wartezeit, typischerweise von mehreren Wochen einzuhalten. Erst nachdem sämtliche vereisten Stellen der Haut 3 vollständig ausgeheilt sind und erst nachdem eine Krustenbildung abgeschlossen und entsprechende mit Farbpigmenten versehene Krusten von der Haut 3 abgefallen sind, kann die Prozedur, bzw. das kosmetische Verfahren wiederholt werden.

Hierzu werden jedoch im Schritt 108 andere Flächensegmente ausgewählt. Dies kann beispielsweise durch einen vorgegebenen Versatz der Maske 20 gegenüber ihrer ursprünglichen Position erfolgen. Danach wird das Verfahren mit den Schritten 102, 104 und 106 erneut durchgeführt. Abhängig vom Erfolg der einzelnen Behandlungen ist nach entsprechenden Wartezeiten das gesamte Verfahren erneut durchzuführen, bis letzten Endes sämtliche Farbpigmente aus der Haut 3 beseitigt wurden.

Es ist ferner auch denkbar, dass einzelne Tätowierungen ohne eine vorab erfolgende Segmentierung mit nur einer einzigen vergleichsweise großflächigen Vereisung entfernt werden. Derartige Tätowierungen dürfen jedoch nicht größer als 1 cm oder 1,5 cm sein.

Das Öffnen oder Abtragen der Epidermis erfolgt typischerweise für sämtliche durch die Maske 20 vorgegebenen Segmente 10.1, 10.2, 10.3, 11.1, 11.2, 11.3 der Haut 3, sofern betroffene Flächensegmente im Bereich der Tätowierung 10 liegen. Auch die nachfolgende Vereisung wird nacheinander für sämtliche Flächensegmente 10.1, 10.2, 10.3, 11.1, 11.2, 11.3 durchgeführt. Eine Krustenbildung erfolgt typischerweise nach wenigen Stunden oder Tagen. Binnen drei Wochen beginnt sich die Kruste zu lösen. Eine Wiederholung der gesamten Prozedur sollte erst hiernach erfolgen. Für eine besonders schonende und möglichst narbenfreie Behandlung der Haut ist es empfehlenswert, eine Pause von etwa acht Wochen zwischen zwei Kältebehandlungen einzuhalten.

Der in Fig. 5 nur schematisch und vereinfacht dargestellte Applikator 30 weist an seinem proximalen Ende eine Aufnahme für eine Kartusche 33 auf, welche mit der kryogenen Flüssigkeit gefüllt ist. Die in Form eines Druckbehälters oder einer Druckpatrone ausgestaltete Kartusche 33 kann auch optional von einem abnehmbaren Gehäusedeckel umschlossen sein. Ferner ist der Applikator 30 mit einem Betätigungselement 34 versehen, welches bei entsprechender Betätigung dafür sorgt, dass ein feiner Strahl der kryogenen Flüssigkeit aus der Düse 32 austritt. Das Betätigungselement 34 kann als Taster oder als Wippschalter ausgestaltet sein. Es kann sich insbesondere für eine Betätigung durch den Zeigefinger eines Nutzers eignen, insbesondere wenn der Applikator 30 nach Art eines Schreibstiftes gehalten wird.

Das Gehäuse 31 kann an einem der Düse 32 zugewandten Endabschnitt ein Griffstück mit zumindest einer Griffmulde 35 aufweisen. Der Applikator kann rein druckkraftbetrieben sein. Die auswechselbare Patrone bzw. der Druckbehälter kann hierbei als Druckreservoir dienen, mittels welchem das kryogene Fluid, d.h. die tiefkalte Flüssigkeit oder das tiefkalte Gas mit vergleichsweise hoher Geschwindigkeit aus der Düse 32 austreten kann.

Für den Auftrag des Vereisungsfluids bzw. der kryogenen Flüssigkeit auf die Haut 3 ist vorgesehen, den Applikator 30 in einem Winkel von 70° bis 90°, das heißt annähernd senkrecht zur Ebene der Haut 3 auszurichten. Der Applikator 30 ist dabei in schnellen, kleinen kreisenden Bewegungen nacheinander über die zu behandelnden Flächensegmente 10.1, 11.1 zu führen. Eine etwaige hierbei entstehende Eisschicht ist dabei mittels des Applikatorstrahls permanent zu entfernen, damit die mit den Farbpigmenten versehene Hautschicht, das heißt die Dermis, optimal eingefroren und kältebehandelt werden kann.

Die Behandlungsdauer für jedes Flächensegment 10.1, 11.1 kann sich auch nach Art der dort vorhandenen Tätowierung richten. Bei einer schwachen Schattierung ist es bereits ausreichend, die betroffene Stelle für nur etwa 15 Sekunden zu vereisen. Bei stark gefärbten Hautflächen sollte eine ununterbrochene Anwendungsdauer bei ca. 20 Sekunden liegen.

### Bezugszeichenliste

- 1: Körperteil
- 2: Unterarm
- 3: Haut
- 10: Tätowierung
- 10.1, 10.2, 10.3: Flächensegment
- 11.1, 11.2, 11.3: Flächensegment
- 20: Maske
- 21: Maskenkörper
- 20.1, 21.2: Durchgangsöffnung
- 30: Applikator
- 31: Gehäuse
- 32: Düse
- 33: Kartusche
- 34: Betätigungselement
- 35: Griffmulde
- 40: Dermabrasionsgerät
- 41: Gehäuse
- 42: Aufsatz
- 43: Abrasionswerkzeug

## Patentansprüche

1. Kosmetisches Verfahren zur Entfernung von Tätowierungen (10) der menschlichen oder tierischen Haut (3), wobei ein Vereisungsfluid auf zumindest ein im Bereich der Tätowierung (10) liegendes Flächensegment (10.1, 11.1) von außen auf die Haut (3) aufgebracht wird.

2. Kosmetisches Verfahren nach Anspruch 1, wobei vor einem Aufbringen des Vereisungsfluids auf die Haut (3), das betreffende Flächensegment (10.1, 11.1) der Haut (3) einer kosmetischen Dermabrasion oder Mikrodermabrasion unterzogen wird.

3. Kosmetisches Verfahren nach Anspruch 2, wobei eine oberste Hautschicht mittels der Dermabrasion oder Mikrodermabrasion lokal geöffnet oder bis zu einer maximalen Tiefe von weniger als 0,1 mm oder von weniger als 0,05 mm abgetragen wird.

4. Kosmetisches Verfahren nach einem der vorhergehenden Ansprüche, wobei die maximale Größe des Flächensegmentes (10.1, 11.1) zwischen 5 mm² und 20 mm² beträgt.

5. Kosmetisches Verfahren nach einem der vorhergehenden Ansprüche, wobei ein mit der Tätowierung (10) versehener Hautbereich in mehrere Flächensegmente (10.1, 10.2, 10.3, 11.1, 11.2, 11.3) unterteilt wird, und wobei nur solche Flächensegmente (10.1, 11.1) mit dem Vereisungsfluid beaufschlagt werden, die um einen vorgegebenen Abstand voneinander entfernt sind.

6. Kosmetisches Verfahren nach Anspruch 5, wobei zur Einteilung der Haut (3) in Flächensegmente und zur Applikation des Vereisungsfluids eine auf die Haut (3) auflegbare Maske (20) mit Durchgangsöffnungen (20.1, 21.1) verwendet wird, wobei die Geometrie und Position der Durchgangsöffnungen (20.1, 21.1) mit der Geometrie und der Position der zu behandelnden Flächensegmente (10.1, 11.1) der Haut (3) übereinstimmen.

7. Kosmetisches Verfahren nach einem der vorhergehenden Ansprüche, wobei das Vereisungsfluid in Form einer kryogenen Flüssigkeit, eines kryogenen Gases oder in Form eines kryogenen Flüssigkeits-Gasgemischs auf das zumindest eine Flächensegment (10.1, 11.2) der Haut (3) aufgebracht wird.

8. Kosmetisches Verfahren nach einem der vorhergehenden Ansprüche, wobei ein Flächensegment (10.1, 11.1) der Haut (3) für eine Zeitdauer von höchstens 15 Sekunden, höchstens 20 Sekunden oder von höchstens 30 Sekunden mit dem Vereisungsfluid beaufschlagt wird.

9. Kosmetisches Verfahren nach einem der vorhergehenden Ansprüche, wobei das Vereisungsfluid in einer kreisenden ununterbrochenen Bewegung auf das gesamte Flächensegment (10.1, 11.1) der Haut (3) verteilt wird.

10. Vorrichtung zur Durchrührung eines kosmetischen Verfahrens zur Entfernung von Tätowierungen (10) der menschlichen oder tierischen Haut (3), mit einem Applikator (30) zum Aufbringen eines Vereisungsfluids auf ein im Bereich der Tätowierung liegendes Flächensegment (10.1, 11.1) von außen auf die Haut (3) und mit einer auf die Haut (3) auflegbaren und mit mehreren Durchgangsöffnungen (20.1, 21.1) versehenen Maske (20).

11. Vorrichtung nach Anspruch 10, ferner mit einem Dermabrasionsgerät (40), um das betreffende Flächensegment (10.1, 11.1) der Haut (3) einer kosmetischen Dermabrasion oder Mikrodermabrasion zu unterziehen.
